# EUROPEAN PATENT APPLICATION

(11) **EP 4 843 608 A1**
(43) Date of publication of application: **25.11.2026**
(21) Application number: 26175231.5
(22) Date of filing: 28.04.2026
(51) Int. Cl.: A61N 1/36, A61B 5/00, A61N 1/372

(54) **INTRAOPERATIVE MAPPING AND TARGETING FOR SPINAL CORD STIMULATION**

(30) Priority: 23.05.2025 US 202563810984 P
(71) Applicant: Boston Scientific Neuromodulation Corporation, Valencia, CA 91355 (US)
(72) Inventor: HADDOCK, Andrew, Los Angeles, CA 90027 (US); MOORE, Lisa, Glendale, CA 91214 (US); STEINKE, G. Karl, Valencia, CA 91354 (US)
(74) Representative: Vossius & Partner Patentanwälte Rechtsanwälte mbB

(57) **Abstract**

Methods and systems for facilitating implantation of electrode leads within a patient's spinal column for spinal cord stimulation (SCS) for treating the patient's pain are described. Transducers are arranged upon the patient's body and used, in conjunction with stimulation applied to the patient's spinal cord, to determine how the spinal stimulation modulates neural signals within the patient's spinal cord. At least some of the transducers may be situated upon the patient's body at locations targeted for pain relief. The system may use the modulation determinations to suggest how to locate the electrode leads to best treat the patient's pain. The system includes a user interface configured to guide the clinician during the lead placement procedure.

## Description

### FIELD OF THE INVENTION

This application relates to Implantable Medical Devices (IMDs) generally, Spinal Cord Stimulators more specifically, and to methods and systems for aiding the implantation of stimulation leads within a patient's spinal column.

### INTRODUCTION

Implantable neurostimulator devices are devices that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will generally focus on the use of the invention within a Spinal Cord Stimulation (SCS) system, such as that disclosed in U.S. Patent 6,516,227.

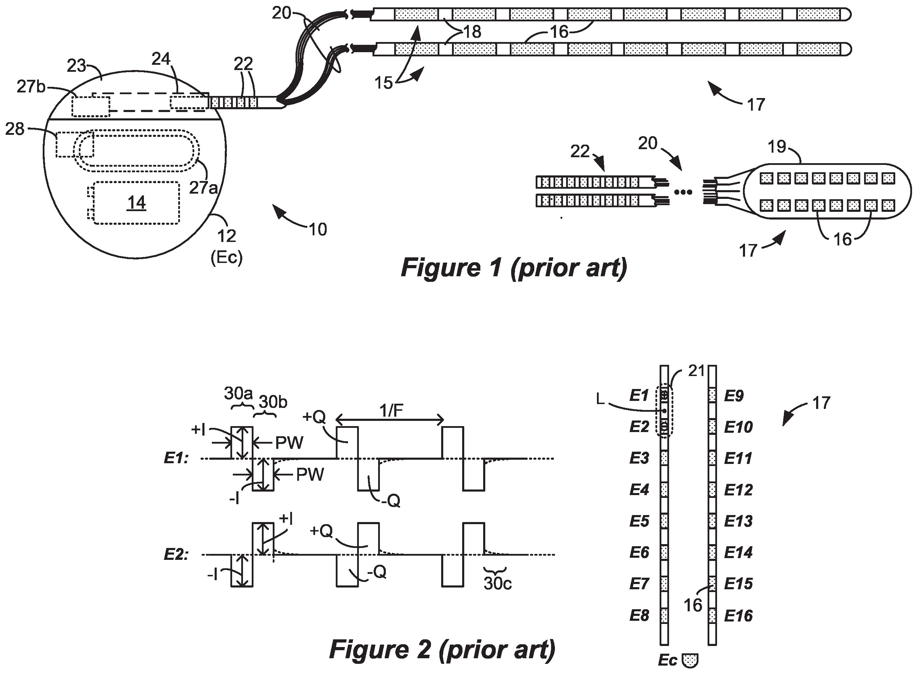

An SCS system typically includes an Implantable Pulse Generator (IPG) 10 shown in Figure 1. The IPG 10 includes a biocompatible device case 12 that holds the circuitry and battery 14 necessary for the IPG to function. The IPG 10 is coupled to electrodes 16 via one or more percutaneous electrode leads 15 that form an electrode array 17. The electrodes 16 are configured to contact a patient's tissue and are carried on a flexible body 18, which also houses the individual lead wires 20 coupled to each electrode 16. The lead wires 20 are also coupled to proximal contacts 22, which are insertable into lead connectors 24 fixed in a header 23 on the IPG 10, which header can comprise an epoxy for example. Once inserted, the proximal contacts 22 connect to header contacts within the lead connectors 24, which are in turn coupled by feedthrough pins through a case feedthrough to stimulation circuitry 28 within the case 12, although these details aren't shown. In the illustrated IPG 10, there are sixteen lead electrodes split between two leads 15, with the header 23 containing a 2x1 array of lead connectors 24. However, the number of leads and electrodes in an IPG is application specific and therefore can vary. The conductive case 12 can also comprise an electrode (Ec). The electrode array 17 can also be provided on a paddle lead 19 comprising a matrix of electrodes 16. The number and arrangement of electrodes of a paddle lead may vary.

IPG 10 can include an antenna 27a allowing it to communicate bi-directionally with a number of external devices, as explained later. The antenna 27a as depicted in Figure 1 is shown as a conductive coil within the case 12, although the coil antenna 27a can also appear in the header 23. When antenna 27a is configured as a coil, communication with external devices preferably occurs using near-field magnetic induction. IPG 10 may also include a Radio-Frequency (RF) antenna 27b. In Figure 1, RF antenna 27b is shown within the header 23, but it may also be within the case 12. RF antenna 27b may comprise a patch, slot, or wire, and may operate as a monopole or dipole. RF antenna 27b preferably communicates using far-field electromagnetic wave, and may operate in accordance with any number of known RF communication standards, such as Bluetooth, Zigbee, WiFi, MICS, and the like.

The goal of SCS therapy is to provide electrical stimulation to the patient's spinal cord to alleviate a patient's symptoms using the electrodes 16. SCS may be used to treat conditions such as chronic back pain, failed back surgery syndrome (FBSS), complex regional pain syndrome (CRPS), peripheral neuropathy such as diabetic peripheral neuropathy (DPN), neuropathic pain, and the like. Stimulation in IPG 10 is typically provided by pulses, as shown in Figure 2. Stimulation parameters typically include the amplitude of the pulses (I; whether current or voltage); the frequency (F) and pulse width (PW) of the pulses; the electrodes 16 (E) activated to provide such stimulation; and the polarity of such active electrodes, i.e., whether active electrodes are to act as anodes (that source current to the tissue) or cathodes (that sink current from the tissue). These stimulation parameters taken together comprise a stimulation program that the IPG 10 can execute to provide therapeutic stimulation to a patient.

In the example of Figure 2, electrode E1 has been selected as an anode (during first phase 30a), and thus provides pulses which source a positive current of amplitude +I to the tissue. Electrode E2 has been selected as a cathode (during first phase 30a), and thus provides pulses which sink a corresponding negative current of amplitude -I from the tissue. This respectively provides an anode pole (+) and a cathode pole (-) at these electrodes, and provides an example of bipolar stimulation, in which these two poles in the electrode array 17 are used to provide stimulation to the tissue. The resulting bipole 21 can be said to have a particular location L in the electrode array 17, which may be differently defined with respect to the anode and cathode. In the example shown this location L is defined as the midway position between the two poles. In another example, the location of the bipole 21 can be defined with respect to only one of the poles, such as the cathode. As explained further below, more than one anode electrode can be active at a given time to establish an anode pole, and more than one cathode electrode may be active at a given time to establish a cathode pole. This allows the location of the anode and cathode poles to be moved or steered in the electrode array in X and Y direction, again as discussed further below.

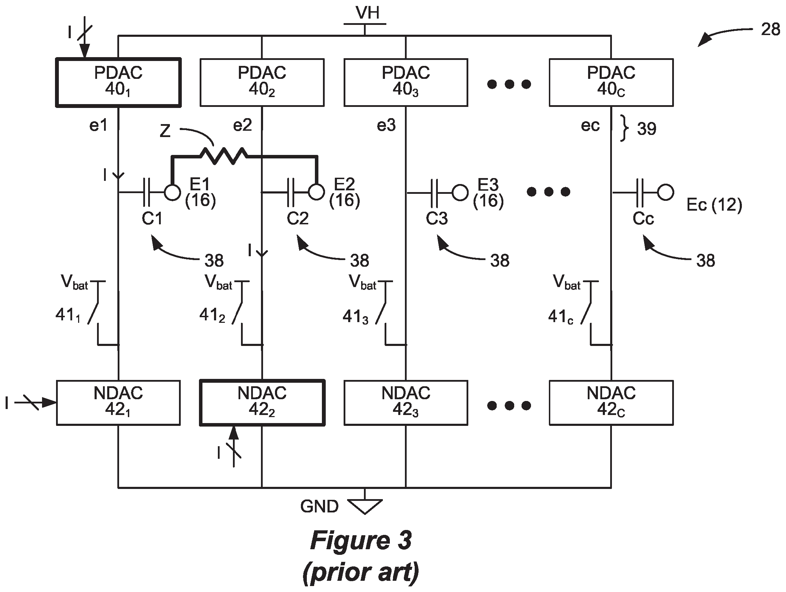

The pulses as shown in Figure 2 are biphasic, comprising a first phase 30a, followed quickly thereafter by a second phase 30b of opposite polarity. As is known, use of a biphasic pulse is useful in active charge recovery. For example, each electrodes' current path to the tissue may include a serially-connected DC-blocking capacitor 38, see Figure 3, which will charge during the first phase 30a and be discharged (charge will be actively recovered) during the second phase 30b. In the example shown, the first and second phases 30a and 30b have the same pulse width (PW) and amplitude (although of opposite polarities), which ensures the same amount of charge is injected and recovered at each electrode during both phases (+Q/-Q). However, the second phase 30b may also be charged balance with the first phase 30a if the integral of the amplitude (i.e., the area) of the two phases are equal in magnitude, as is well known.

IPG 10 includes stimulation circuitry 28 to form prescribed stimulation pulses, and Figure 3 shows an example of such circuitry, which includes one or more current sources 40ᵢ and one or more current sinks 42ᵢ. The sources and sinks 40ᵢ and 42ᵢ can comprise Digital-to-Analog converters (DACs), and may be referred to as PDACs 40ᵢ and NDACs 42ᵢ in accordance with the Positive (sourced, anodic) and Negative (sunk, cathodic) currents they respectively issue. In the example shown, a NDAC/PDAC 40ᵢ/42ᵢ pair is dedicated (hardwired) to a particular electrode node ei 39. Each electrode node ei 39 is connected to an electrode Ei 16 via a DC-blocking capacitor Ci 38, for the reasons explained below. PDACs 40ᵢ and NDACs 42ᵢ can also comprise voltage sources.

Proper control of the PDACs 40ᵢ and NDACs 42ᵢ allows any of the electrodes 16 and the case electrode Ec 12 to act as anodes or cathodes to create a current through a patient's tissue, Z, hopefully with good therapeutic effect. In the example shown, and consistent with the first pulse phase 30a of Figure 2, electrode E1 has been selected as an anode electrode to source current I to the tissue Z and electrode E2 has been selected as a cathode electrode to sink current I from the tissue. Thus PDAC 40₁ and NDAC 42₂ are activated and digitally programmed to produce the desired current, I, with the correct timing (e.g., in accordance with the prescribed frequency F and pulse width PW). During the second phase 30b, the direction of the current through the tissue would be reversed by activating PDACs 40₂ and NDACs 42ᵢ to produce current I. Power for the stimulation circuitry 28 is provided by a compliance voltage VH, as described in further detail in U.S. Patent Application Publication 2013/0289665.

Other stimulation circuitries 28 can also be used in the IPG 10. In an example not shown, a switching matrix can intervene between the one or more PDACs 40ᵢ and the electrode nodes ei 39, and between the one or more NDACs 42ᵢ and the electrode nodes. Switching matrices allow one or more of the PDACs or one or more of the NDACs to be connected to one or more electrode nodes at a given time. Various examples of stimulation circuitries can be found in USPs 6,181,969, 8,606,362, 8,620,436, U.S. Patent Application Publications 2018/0071520 and 2019/0083796.

Much of the stimulation circuitry 28 of Figure 3, including the PDACs 40ᵢ and NDACs 42ᵢ, the switch matrices (if present), and the electrode nodes ei 39 can be integrated on one or more Application Specific Integrated Circuits (ASICs), as described in U.S. Patent Application Publications 2012/0095529, 2012/0092031, and 2012/0095519. As explained in these references, ASIC(s) may also contain other circuitry useful in the IPG 10, such as telemetry circuitry (for interfacing off chip with telemetry antennas 27a and/or 27b), circuitry for generating the compliance voltage VH, various measurement circuits, etc.

Also shown in Figure 3 are DC-blocking capacitors Ci 38 placed in series in the electrode current paths between each of the electrode nodes ei 39 and the electrodes Ei 16 (including the case electrode Ec 12). The DC-blocking capacitors 38 act as a safety measure to prevent DC current injection into the patient, as could occur for example if there is a circuit fault in the stimulation circuitry 28. The DC-blocking capacitors 38 are typically provided off-chip (off of the ASIC(s)), and instead may be provided in or on a circuit board in the IPG 10 used to integrate its various components, as explained in U.S. Patent Application Publication 2015/0157861.

The stimulation circuitry 28 can include passive recovery switches 41ᵢ, which are described further in U.S. Patent Application Publications 2018/0071527 and 2018/0140831. Passive recovery switches 41ᵢ may be closed to passively recover any charge remaining on the DC-blocking capacitors Ci 38 after issuance of a pulse (e.g., after second pulse phase 30b during phase 30c as shown in Fig. 2) to recover charge without actively driving a current using the DAC circuitry, as shown during duration 30c. This is beneficial, because active charge recovery using biphasic pulses as described earlier may not operate perfectly, and therefore some small amount of residual charge may still require passive recovery. Again, passive charge recovery is well known and not further described. Although not shown, the stimulation pulses may also be monophasic comprising single actively-driven phases (e.g., 30a), each followed by passive charge recovery (e.g., 30c).

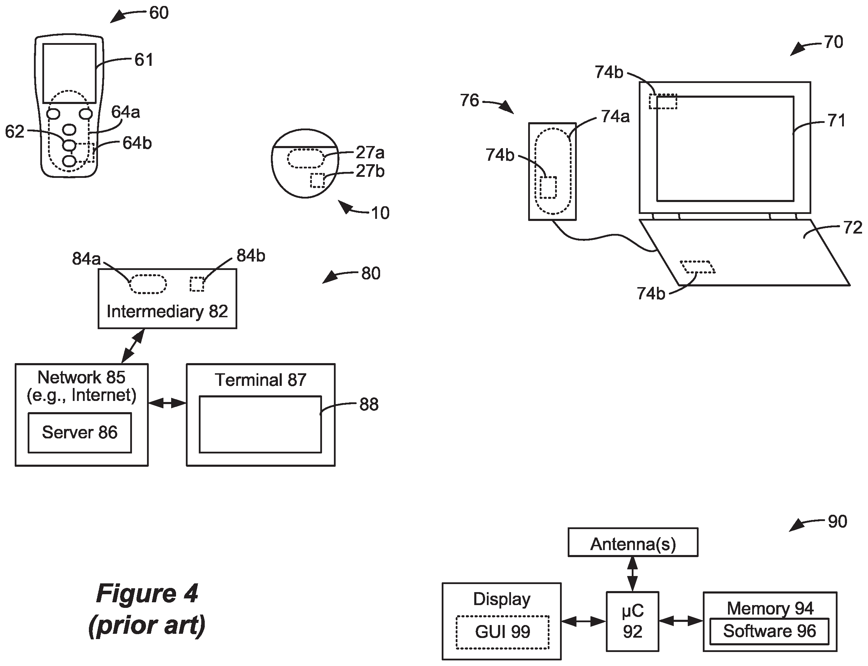

Figure 4 shows various external systems 60, 70, and 80 that can wirelessly communicate data with the IPG 10. Such systems can be used to wirelessly transmit a stimulation program to the IPG 10-that is, to program its stimulation circuitry 28 to produce stimulation with desired amplitudes and timings as described earlier. Such systems may also be used to adjust one or more stimulation parameters of a stimulation program that the IPG 10 is currently executing, and/or to wirelessly receive information from the IPG 10, such as various status information and measurements, etc.

External Controller (EC) 60 can be as described in U.S. Patent Application Publication 2015/0080982 for example, and may comprise a portable, hand-held controller dedicated to work with the IPG 10. External controller 60 may also comprise a general-purpose mobile electronics device such as a mobile phone which has been programmed with a Medical Device Application (MDA) allowing it to work as a wireless controller for the IPG 10, as described in U.S. Patent Application Publication 2015/0231402. External controller 60 includes a display 61 and a means for entering commands, such as buttons 62 or selectable graphical icons provided on the display 61. The external controller 60's user interface enables a patient to adjust stimulation parameters, although it may have limited functionality when compared to systems 70 and 80, described shortly. The external controller 60 can have one or more antennas capable of communicating with a compatible antenna 27a and/or 27b in the IPG 10, such as a near-field magnetic-induction coil antenna 64a and/or a far-field RF antenna 64b.

Clinician programmer (CP) 70 is described further in U.S. Patent Application Publication 2015/0360038, and can comprise a computing device such as a desktop, laptop, or notebook computer, a tablet, a mobile smart phone, a Personal Data Assistant (PDA)-type mobile computing device, etc. In Figure 4, the computing device is shown as a laptop computer that includes typical computer user interface means such as a display 71, buttons 72, as well as other user-interface devices such as a mouse, a keyboard, speakers, a stylus, a printer, etc., not all of which are shown for convenience. Also shown in Figure 4 are accessory devices for the clinician programmer 70 that are usually specific to its operation as a stimulation controller. A communication "wand" 76 coupleable to suitable ports on the computing device can include an IPG-compliant antenna such as a coil antenna 74a or an RF antenna 74b. The computing device itself may also include one or more RF antennas 74b. The clinician programmer 70 can also communicate with other devices and networks, such as the Internet, either wirelessly or via a wired link provided at an Ethernet or network port.

External system 80 comprises another means of communicating with and controlling the IPG 10 via a network 85 which can include the Internet. The network 85 can include a server 86 programmed with IPG communication and control functionality, and may include other communication networks or links such as WiFi, cellular or land-line phone links, etc. The network 85 ultimately connects to an intermediary device 82 having antennas suitable for communication with the IPG's antenna, such as a near-field magnetic-induction coil antenna 84a and/or a far-field RF antenna 84b. Intermediary device 82 may be located generally proximate to the IPG 10. Network 85 can be accessed by any user terminal 87, which typically comprises a computer device associated with a display 88. External system 80 allows a remote user at terminal 87 to communicate with and control the IPG 10 via the intermediary device 82.

Figure 4 also shows circuitry 90 involved in any of external systems 60, 70, or 80. Such circuitry can include control circuitry 92, which can comprise any number of devices such as one or more microprocessors, microcomputers, FPGAs, DSPs, other digital logic structures, etc., which are capable of executing programs in a computing device. Such control circuitry 92 may contain or coupled with memory 94 which can store external system software 96 for controlling and communicating with the IPG 10, and for rendering a Graphical User Interface (GUI) 99 on a display (61, 71, 88) associated with the external system. Memory 94 may comprise solid state, optical, or magnetic memories for example capable of storing the software 96 in a non-transitory fashion. In external system 80, the external system software 96 would likely reside in the server 86, while the control circuitry 92 could be present in either or both the server 86 or the terminal 87.

In a SCS application, the electrode array 17 (leads 15 or paddle 19) are typically implanted proximate to the dura within a patient's spinal column such that the electrodes are located on the right and left sides of the spinal cord midline, for example. The proximal electrodes 22 and lead wires 20 are tunneled through the patient's tissue to a distant location such as the buttocks where the IPG case 12 is implanted, at which point they are coupled to the lead connectors 24. The implantation of the electrode leads may involve the patient undergoing a laminectomy (removal of part of the patient's, the bony arches that cover the spine), especially if paddle leads are used.

Because of the invasiveness of the surgery for implanting the leads and the IPG, a patient that is a candidate for SCS typically undergoes a trial stimulation phase prior to committing to implantation of a permanent SCS system. During the trial phase one or more trial leads are implanted within the spinal column. The implantation of the trial leads involves inserting the trial leads into the epidural space while the patient is typically under local anesthesia. The proximal ends of the lead wires are left protruding from the patient's body and are connected to an External Trial Stimulator (ETS), which is a device that is functionally similar to the IPG 10 but that is configured to be worn outside the patient's body, either in a case or by securing the ETS to the patient's body using tape. See, e.g., 9,259,574, disclosing a design for an ETS. The ETS typically provides similar functionality to the IPG described above. It is configured with similar stimulation circuitry and telemetry capabilities for communicating with external devices, such as a CP, EC, or the like. The ETS is worn externally by the patient for a short while (e.g., two weeks), which allows the patient and his clinician to experiment with different stimulation parameters to try and find a stimulation program that alleviates the patient's symptoms (e.g., pain). If external trial stimulation proves successful, trial lead(s) are explanted, and a permanent IPG 10 and electrode array 17 are implanted as described above; if unsuccessful, the trial lead(s) are simply explanted.

### SUMMARY

This Summary includes examples that provide an overview of some of the teachings of the present disclosure. For example, disclosed herein is a system for assisting the surgical implantation of an electrode array in the spinal column of a patient for delivering therapeutic stimulation to the patient's spinal cord to treat pain in the patient, wherein the electrode array comprises a plurality of spinal electrodes, the system comprising: a user interface, control circuitry configured to: receive an indication of locations of one or more peripheral transducers placed at peripheral locations on the patient's body, receive one or more acceptance criteria, cause a predetermined one or more of the plurality of spinal electrodes to provide test spinal stimulation to the patient's spinal cord, receive one or more signals indicative of neural activity, wherein the neural activity is either evoked or recorded using the one or more peripheral transducers, determine a modulation of the neural activity by the test spinal stimulation, use the modulation to determine if the acceptance criteria is met, if the acceptance criteria is met, cause the user interface to display an indication that the acceptance criteria is met, if the acceptance criteria is not met, use the modulation to determine a suggested movement of the electrode array and cause the user interface to display an indication of the suggested movement. According to some embodiments, the locations on the patient's body correspond to body locations targeted for pain relief. According to some embodiments, the one or more acceptance criteria comprises a desired location within the electrode array selected for delivering the therapeutic stimulation. According to some embodiments, the one or more acceptance criteria comprises an acceptable coverage of body locations targeted for the therapeutic stimulation. According to some embodiments, the one or more acceptance criteria comprises an acceptable coverage of dermatomes targeted for the therapeutic stimulation. According to some embodiments, the neural activity comprises potentials evoked by the test spinal stimulation and recorded by the one or more peripheral transducers. According to some embodiments, the one or more peripheral transducers are locatable at one or more body locations of the patient targeted for the therapeutic stimulation and comprise one or more of needle electrodes, electromyography (EMG) electrodes, transcutaneous electrodes, and/or dermal electrodes. According to some embodiments, the neural activity comprises motor evoked potentials (MEPs). According to some embodiments, the MEPs are evoked by stimulating the patient's motor cortex and recorded by the one or more peripheral transducers. According to some embodiments, determining a modulation of the neural activity by the test spinal stimulation comprises: recording first MEPs in the absence of the test spinal stimulation, recording second MEPs in the presence of the test spinal stimulation, and determining a difference between the first and second MEPs. According to some embodiments, the one or more peripheral transducers are locatable at one or more body locations of the patient targeted for the therapeutic stimulation and comprise one or more of needle electrodes, electromyography (EMG) electrodes, transcutaneous electrodes, and/or dermal electrodes. According to some embodiments, the neural activity comprises sensory evoked potentials (SEPs). According to some embodiments, the SEPs are evoked by stimulation provided by the one or more peripheral transducers and recorded at the patient's somatosensory cortex. According to some embodiments, determining a modulation of the neural activity by the test spinal stimulation comprises: recording first SEPs in the absence of the test spinal stimulation, recording second SEPs in the presence of the test spinal stimulation, and determining a difference between the first and second SEPs. According to some embodiments, the one or more peripheral transducers are locatable at one or more body locations of the patient targeted for the therapeutic stimulation and comprise one or more of needle electrodes, electromyography (EMG) electrodes, transcutaneous electrodes, and/or dermal electrodes. According to some embodiments, the neural activity comprises afferent neural signals evoked by stimulation provided by the one or more peripheral transducers and recorded using one or more of the spinal electrodes. According to some embodiments, the one or more peripheral transducers are locatable at one or more body locations of the patient targeted for the therapeutic stimulation and comprise one or more of needle electrodes, electromyography (EMG) electrodes, transcutaneous electrodes, and/or dermal electrodes. According to some embodiments, displaying an indication of the suggested movement comprises displaying an image indicative of the present location of the electrode array and an indication of a position the electrode array will occupy following the suggested array movement. According to some embodiments, the image indicative of the present location comprises a fluoroscopic image. According to some embodiments, displaying an indication of the suggested movement comprises displaying a distance and direction to move the array. According to some embodiments, displaying an indication of the suggested movement comprises displaying an instruction to move the array to a particular location with respect to an indicated anatomical landmark. According to some embodiments, the indicated anatomical landmark comprises a vertebrae or a subcomponent of a vertebrae. According to some embodiments, the control circuitry is configured to cause the user interface to display an indication of an initial location for the electrode array. According to some embodiments, the control circuitry is configured to receive one or more parameters derived from a previously placed trial electrode array. According to some embodiments, the one or more parameters derived from the previously implanted trial electrode array comprises a fluoroscopic image of the previously implanted trial electrode array. According to some embodiments, the one or more parameters derived from the previously implanted trial electrode array comprises one or more indications of electrodes of the trial electrode array used to deliver therapeutic stimulation to the patient during a trial stimulation phase. According to some embodiments, the control circuitry is configured to cause the user interface to display indicia of body locations where the peripheral transducers should be placed. According to some embodiments, the control circuitry is configured to cause the system to project indicia of body locations where the peripheral transducers should be placed onto the patient's body. According to some embodiments, the control circuitry is configured to cause the system to display indicia of body locations where the peripheral transducers should be placed on an external screen. According to some embodiments, the system is configured to: receive one or more user pain inputs indicating locations on the patient's body that the patient feels pain, digitize the pain inputs, and use the digitized inputs to generate the indicia of body locations where the peripheral transducers should be placed. According to some embodiments, the system is configured to: display one or more body maps comprising selectable body map regions, receive indications of one or more selected body map regions corresponding to body locations where the patient feels the pain, use a translation algorithm to determine mediolateral locations and dermatomes where the patient feels the pain based on the selected body map regions, and use the mediolateral locations and dermatomes to determine the indicia of body locations where the peripheral transducers should be placed.

Also disclosed herein is a method for assisting the surgical implantation of an electrode array in the spinal column of a patient for delivering therapeutic stimulation to the patient's spinal cord to treat pain in the patient, wherein the electrode array comprises a plurality of spinal electrodes, the method comprising: receiving indications of locations of one or more peripheral transducers placed at peripheral locations on the patient's body, receiving one or more acceptance criteria, causing a predetermined one or more of the plurality of spinal electrodes to provide test spinal stimulation to the patient's spinal cord, receiving one or more signals indicative of neural activity, wherein the neural activity is either evoked or recorded using the one or more peripheral transducers, determining a modulation of the neural activity by the test spinal stimulation, using the modulation to determine if the acceptance criteria is met, if the acceptance criteria is met, causing a user interface to display an indication that the acceptance criteria is met, if the acceptance criteria is not met, using the modulation to determine a suggested movement of the electrode array and causing the user interface to display an indication of the suggested movement. According to some embodiments, the locations on the patient's body correspond to body locations targeted for pain relief. According to some embodiments, the one or more acceptance criteria comprises a desired location within the electrode array selected for delivering the therapeutic stimulation. According to some embodiments, the one or more acceptance criteria comprises an acceptable coverage of body locations targeted for the therapeutic stimulation. According to some embodiments, the one or more acceptance criteria comprises an acceptable coverage of dermatomes targeted for the therapeutic stimulation. According to some embodiments, the neural activity comprises potentials evoked by the test spinal stimulation and recorded by the one or more peripheral transducers. According to some embodiments, the one or more peripheral transducers are locatable at one or more body locations of the patient targeted for the therapeutic stimulation and comprise one or more of needle electrodes, electromyography (EMG) electrodes, transcutaneous electrodes, and/or dermal electrodes. According to some embodiments, the neural activity comprises motor evoked potentials (MEPs). According to some embodiments, the MEPs are evoked by stimulating the patient's motor cortex and recorded by the one or more peripheral transducers. According to some embodiments, determining a modulation of the neural activity by the test spinal stimulation comprises: recording first MEPs in the absence of the test spinal stimulation, recording second MEPs in the presence of the test spinal stimulation, and determining a difference between the first and second MEPs. According to some embodiments, the one or more peripheral transducers are locatable at one or more body locations of the patient targeted for the therapeutic stimulation and comprise one or more of needle electrodes, electromyography (EMG) electrodes, transcutaneous electrodes, and/or dermal electrodes. According to some embodiments, the neural activity comprises sensory evoked potentials (SEPs). According to some embodiments, the SEPs are evoked by stimulation provided by the one or more peripheral transducers and recorded at the patient's somatosensory cortex. According to some embodiments, determining a modulation of the neural activity by the test spinal stimulation comprises: recording first SEPs in the absence of the test spinal stimulation, recording second SEPs in the presence of the test spinal stimulation, and determining a difference between the first and second SEPs. According to some embodiments, the one or more peripheral transducers are locatable at one or more body locations of the patient targeted for the therapeutic stimulation and comprise one or more of needle electrodes, electromyography (EMG) electrodes, transcutaneous electrodes, and/or dermal electrodes. According to some embodiments, the neural activity comprises afferent neural signals evoked by stimulation provided by the one or more peripheral transducers and recorded using one or more of the spinal electrodes. According to some embodiments, the one or more peripheral transducers are locatable at one or more body locations of the patient targeted for the therapeutic stimulation and comprise one or more of needle electrodes, electromyography (EMG) electrodes, transcutaneous electrodes, and/or dermal electrodes. According to some embodiments, displaying an indication of the suggested movement comprises displaying an image indicative of the present location of the electrode array and an indication of a position the electrode array will occupy following the suggested array movement. According to some embodiments, the image indicative of the present location comprises a fluoroscopic image. According to some embodiments, displaying an indication of the suggested movement comprises displaying a distance and direction to move the array. According to some embodiments, displaying an indication of the suggested movement comprises displaying an instruction to move the array to a particular location with respect to an indicated anatomical landmark. According to some embodiments, the indicated anatomical landmark comprises a vertebrae or a subcomponent of a vertebrae. According to some embodiments, the method comprises causing the user interface to display an indication of an initial location for the electrode array. According to some embodiments, the method comprises receiving one or more parameters derived from a previously placed trial electrode array. According to some embodiments, the one or more parameters derived from the previously implanted trial electrode array comprises a fluoroscopic image of the previously implanted trial electrode array. According to some embodiments, the one or more parameters derived from the previously implanted trial electrode array comprises one or more indications of electrodes of the trial electrode array used to deliver therapeutic stimulation to the patient during a trial stimulation phase. According to some embodiments, the method comprises causing the user interface to display indicia of body locations where the peripheral transducers should be placed. According to some embodiments, the method comprises projecting the indicia of body locations where the peripheral transducers should be placed onto the patient's body. According to some embodiments, the method comprises displaying the indicia of body locations where the peripheral transducers should be placed on an external screen. According to some embodiments, the method comprises: receiving one or more user pain inputs indicating locations on the patient's body that the patient feels pain, digitizing the pain inputs, and using the digitized inputs to generate the indicia of body locations where the peripheral transducers should be placed. According to some embodiments, the method comprises displaying one or more body maps comprising selectable body map regions, receiving indications of one or more selected body map regions corresponding to body locations where the patient feels the pain, using a translation algorithm to determine mediolateral locations and dermatomes where the patient feels the pain based on the selected body map regions, and using the mediolateral locations and dermatomes to determine the indicia of body locations where the peripheral transducers should be placed.

Also disclosed herein is a method for assisting the surgical implantation of an electrode array in the spinal column of a patient for delivering therapeutic stimulation to the patient's spinal cord to treat pain in the patient, wherein the electrode array comprises a plurality of spinal electrodes, the method comprising: placing the electrode array at a first location within the patient's spinal column, causing a predetermined one or more of the plurality of spinal electrodes to provide test spinal stimulation to the patient's spinal cord, using electrical stimulation of the patient's motor cortex to evoke motor evoked potentials (MEPs), using one or more electrodes placed on the patient's body at locations targeted for pain relief to record the MEPs, wherein the MEPs are recorded in the presence and in the absence of the test spinal stimulation, using the MEPs recorded in the presence and in the absence of the test spinal stimulation to determine a modulation of the MEPs by the test spinal stimulation, using the modulation of the MEPs to suggest a movement of the electrode array, and causing a user interface to display an indication of the suggested movement.

Also disclosed herein is a system for assisting the surgical implantation of an electrode array in the spinal column of a patient for delivering therapeutic stimulation to the patient's spinal cord to treat pain in the patient, wherein the electrode array comprises a plurality of spinal electrodes, the system comprising: a user interface, control circuitry configured to: receive an indication of a first location of the electrode array within the patient's spinal column, cause a predetermined one or more of the plurality of spinal electrodes to provide test spinal stimulation to the patient's spinal cord, use electrical stimulation of the patient's motor cortex to evoke motor evoked potentials (MEPs), use one or more electrodes placed on the patient's body at locations targeted for pain relief to record the MEPs, wherein the MEPs are recorded in the presence and in the absence of the test spinal stimulation, use the MEPs recorded in the presence and in the absence of the test spinal stimulation to determine a modulation of the MEPs by the test spinal stimulation, use the modulation of the MEPs to suggest a movement of the electrode array, and cause the user interface to display an indication of the suggested movement.

Also disclosed herein is a method for assisting the surgical implantation of an electrode array in the spinal column of a patient for delivering therapeutic stimulation to the patient's spinal cord to treat pain in the patient, wherein the electrode array comprises a plurality of spinal electrodes, the method comprising: placing the electrode array at a first location within the patient's spinal column, causing a predetermined one or more of the plurality of spinal electrodes to provide test spinal stimulation to the patient's spinal cord, cause one or more electrodes placed on the patient's body at locations targeted for pain relief to evoke sensory evoked potentials (SEPs), cause one or more transducers to record the SEPs at the patient's somatosensory cortex, wherein the SEPs are recorded in the presence and in the absence of the test spinal stimulation, using the SEPs recorded in the presence and in the absence of the test spinal stimulation to determine a modulation of the SEPs by the test spinal stimulation, using the modulation of the SEPs to suggest a movement of the electrode array, and causing a user interface to display an indication of the suggested movement.

Also disclosed herein s a system for assisting the surgical implantation of an electrode array in the spinal column of a patient for delivering therapeutic stimulation to the patient's spinal cord to treat pain in the patient, wherein the electrode array comprises a plurality of spinal electrodes, the system comprising: a user interface, control circuitry configured to: receive an indication of a first location of the electrode array within the patient's spinal column, cause a predetermined one or more of the plurality of spinal electrodes to provide test spinal stimulation to the patient's spinal cord, cause one or more electrodes placed on the patient's body at locations targeted for pain relief to stimulate the patient to evoke sensory evoked potentials (SEPs), cause one or more transducers to record the SEPs at the patient's somatosensory cortex, wherein the SEPs are recorded in the presence and in the absence of the test spinal stimulation, use the SEPs recorded in the presence and in the absence of the test spinal stimulation to determine a modulation of the MEPs by the test spinal stimulation, use the modulation of the SEPs to suggest a movement of the electrode array, and cause the user interface to display an indication of the suggested movement.

The invention may also reside in the methods performed by the above-described systems, graphical user interfaces for facilitating those methods, and non-transitory computer readable media containing instructions for programming a system and/or an external device (via its control circuitry) for carrying out the methods, a programmed implantable pulse generator (IPG) or external trial stimulator (ETS), external pulse generator (EPG), (via their respective control circuitry) for carrying out the above methods, a system including a programmed external device, such as a clinician's programmer and auxiliary devices that interact with an IPG or ETS for carrying out the above methods, or as a computer-readable media for carrying out the above methods stored in an external device or IPG or ETS.

### BRIEF DESCRIPTION OF THE DRAWINGS

Figure 1 shows a Spinal Cord Stimulation (SCS) Implantable Pulse Generator (IPG) and its electrode array, in accordance with the prior art.
Figure 2 shows an example of stimulation pulses producible by the IPG, in accordance with the prior art.
Figure 3 shows stimulation circuitry useable in an IPG, in accordance with the prior art.
Figure 4 shows various external devices capable of communicating with and programming stimulation in an IPG, in accordance with the prior art.
Figure 5 shows a system for guiding intraoperative placement of a spinal electrode array.
Figure 6 shows components of a system for guiding intraoperative placement of a spinal electrode array.
Figure 7 shows circuitry for sensing neural potentials.
Figure 8 shows circuitry for an interface for peripheral transducers.
Figure 9 shows a logical overview of some embodiments of a guidance system and method for guiding intraoperative lead placement.
Figure 10 shows an embodiment of a graphical user interface (GUI) for selecting pain locations.
Figure 11 shows an embodiment of translating selected body map regions to dermatomes.
Figures 12A-12D show examples of physiological measurements for guiding the placement of a spinal electrode array.
Figure 13 shows examples of electromyography recordings evoked by different stimulation electrodes within a spinal electrode array.
Figure 14 shows an example of a GUI for instructing a clinician how to reposition a spinal electrode array.

### DETAILED DESCRIPTION

When an SCS system is implanted in a patient, the clinician or surgeon will seek to implant the electrode lead(s) (e.g., 15 or 19, Fig. 1) at a position within the patient's spinal column where the electrodes 16 will be capable of providing electrical stimulation that modulates the appropriate neural elements within the spinal cord for treating the patient's symptoms. In other words, the leads should be placed at a position where the appropriate neural elements can be targeted for stimulation. When trial electrode leads are being implanted, the patient is typically awake and can therefore facilitate the clinician's efforts to appropriately position the leads. The clinician can place the lead in a trial location and then apply stimulation to one or more of the electrodes. Stimulation of an appropriate amplitude will evoke sensations (referred to as paresthesia), such as tingling, that the patient will perceive at a location on their body (specifically, a dermatome) innervated by nerves being modulated by the stimulation. Thus, the patient can help guide the clinician as the clinician seeks to find a lead placement where stimulation at some of the electrodes results in an overlap of the paresthesia with the patient's pain, for example.

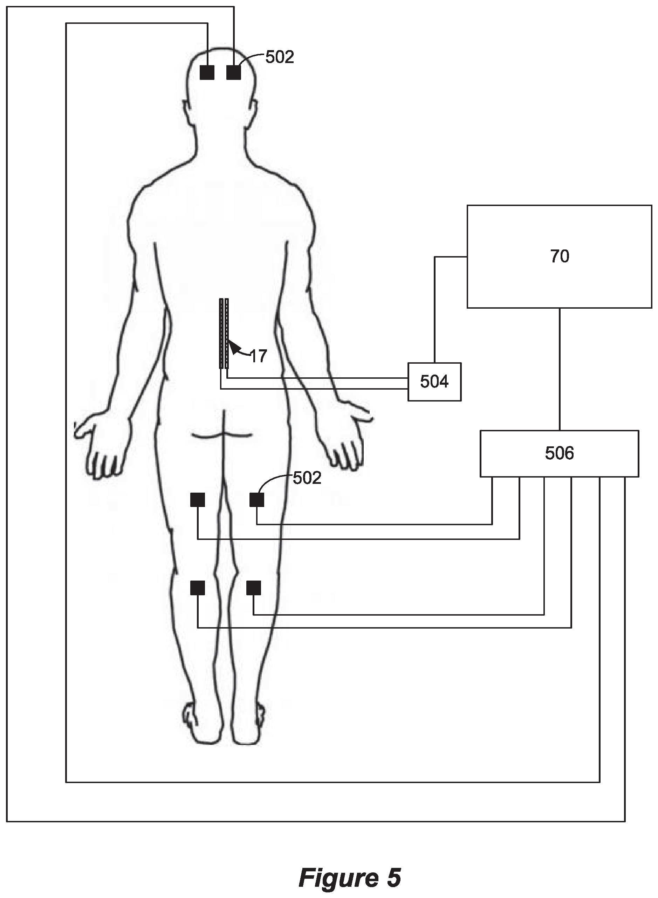

But during the implantation of permanent leads the patient is typically anesthetized at a level that renders them incapable of providing such feedback. Aspects of this disclosure relate to methods and systems to facilitate and guide intraoperative electrode lead placement within a patient's spinal column while a patient is under anesthesia. As shown in Figure 5, embodiments of the disclosed systems use electrical transducers 502 that are positioned outside the patient's spinal column and thus referred to herein as "non-spinal transducers." The non-spinal transducers may be located at the patient's periphery, on the patient's trunk, and/or at the patient's head, depending on the nature of the signals being evoked/recorded using the transducers. In various embodiments, the transducers may be configured to either impart electrical signals to, or sense electrical signals from, the patient's neuromuscular system with the goal of measuring how stimulation provided to the patient's spinal cord using electrodes of the electrode array 17 modulates electrical signals within the spinal cord. The system processes these measurements and uses them to provide recommendations to the clinician as they seek to properly locate the electrode array within the patient's spinal column. As explained above, the electrode array 17 may comprise a plurality of electrodes 16 (Fig. 1) configured upon one or more leads (e.g., 15 or 19, Fig. 1). The array may be connected to an ETS 504, which may comprise stimulation circuitry 28 (Fig. 3) as described above. The transducers 502 may be connected to a transducer interface 506. The transducer interface 506 and the ETS 504 may be operatively connected to a computing device, such as a CP 70.

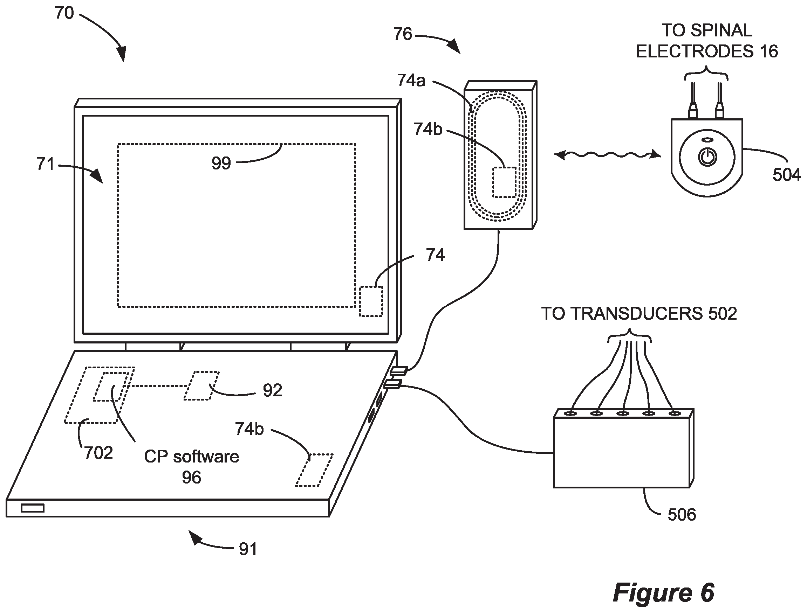

Figure 6 shows more details of the ETS 504, transducer interface 506, and CP 70. The CP 70 is generally as described above and the same reference numbers refer to the same components described above. The CP software 96 configured to execute the steps and functionality described herein may be stored within nonvolatile memory 702 of the CP. The functionality of the ETS 504 is generally as described above. The ETS may communicate with the CP via a coil antenna 74a and or an RF antenna 74b as described above. In other embodiments, not shown, the ETS may communicate with the CP via a hardwired connection.

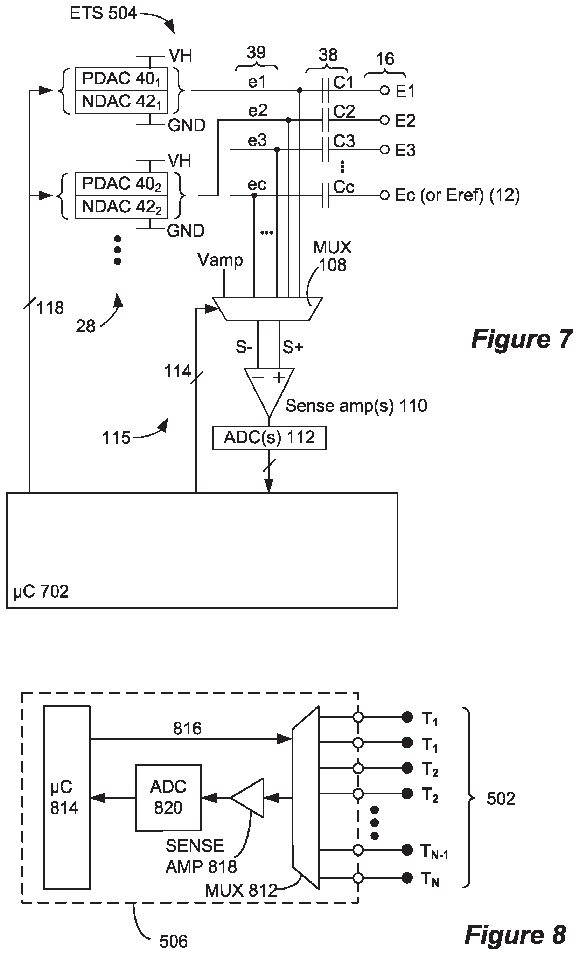

In some embodiments the ETS may be configured with sensing circuitry that provides the ability to sense and record electrical signals in the patient's spinal cord using one or more electrodes 16 of the electrode array 17 as sensing electrodes. Figure 7 illustrates an example embodiment of an ETS 504 configured with sensing circuitry whereby one or more of the electrodes 16 can be used to sense electrical signals, e.g., biopotentials from the patient's tissue. In this regard, each electrode node 39 can be coupled to a sense amp circuit 110. Under control by bus 114, a multiplexer 108 can select one or more electrodes to operate as sensing electrodes (S+, S-) by coupling the electrode(s) to the sense amps circuit 110 at a given time, for example. Although only one multiplexer 108 and sense amp circuit 110 are shown in Figure 7, there could be more than one. For example, there can be four multiplexer 108/sense amp circuit 110 pairs each operable within one of four timing channels supported by the ETS 504 to provide stimulation. The sensed signals output by the sense amp circuitry are preferably converted to digital signals by one or more Analog-to-Digital converters (ADC(s)) 112, which may sample the output of the sense amp circuit 110 at 50 kHz for example. The ADC(s) 112 may also reside within the ETS's control circuitry 702, particularly if the control circuitry has A/D inputs. In some embodiments, the ETS's control circuitry may comprise a microcontroller, or which may be formed in whole or in part in one or more Application Specific Integrated Circuits (ASICs). Multiplexer 108 can also provide a fixed reference voltage, Vamp, to the sense amp circuit 110, as is useful in a single-ended sensing mode (i.e., to set S- to Vamp). As mentioned above, the ETS 504 (via its control circuitry 702) may be controlled by the CP 70, via its control circuitry 92.

Referring again to Figure 6, the transducer interface 506 may be configured to receive electrical signals from, and generate electrical signals for, the transducers 502. Figure 8 shows an example of an embodiment of a transducer interface 506. In the illustrated example, the transducers 502 (T1-TN) are coupled with a multiplexer 812. The multiplexer passes the signals from one or more of the transducers to a sense amplifier 818 based on signals it receives from control circuitry 814 over a bus 816. The analog signals received from the sense amplifier 818 may be digitized using an analog-to-digital converter 820 and provided to the microcontroller 814. Though not shown, the transducer interface may also be configured to provide stimulating electrical signals to the transducers 502, whereby the transducers can electrically stimulate the patient's tissue. In some embodiments, the transducer interface's control circuitry may comprise a microcontroller, or which may be formed in whole or in part in one or more Application Specific Integrated Circuits (ASICs). As mentioned above, the transducer interface (via its control circuitry 702) may be controlled by the CP 70, via its control circuitry 92. Other configurations of a transducer interface may be used with the disclosed methods and systems. For example, as will be explained in more detail below, embodiments of the disclosed methods may involve the recording of motor evoked potentials (MEPs), electromyography (EMG), sensory evoked potentials (SEPs), and the like. Any transducers, transducer interfaces or related systems known in the art for making such measurements may be used within the context of the disclosed methods and systems.

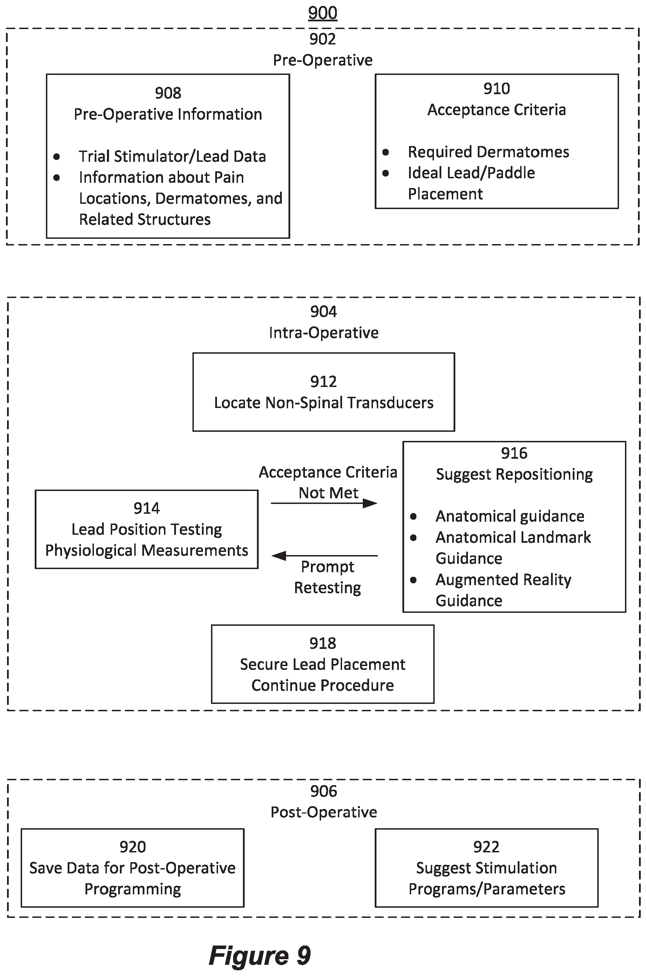

Figure 9 illustrates a logical overview 900 of some embodiments of the guidance systems and methods described herein for guiding intraoperative lead placement. Aspects of the systems and methods may be logically categorized as pre-operative aspects 902, intra-operative aspects 904, and post-operative aspects 906. It should be appreciated that these categories are not limiting and there may be overlap between categories. At a high level, the disclosed methods and systems use pre-operative knowledge to inform physiological measurements that can be evaluated during the lead placement operation to guide the placement of the stimulation leads such that certain acceptance criteria are met. Data collected during the lead placement operation can be saved and used to inform post-operative stimulation programming.

Prior to implanting the stimulation leads within the patient, the clinician will need to gather pre-operative information 908 that will inform the objectives of the lead implantation procedure, as well as the physiological measurements that will be acquired and evaluated during the implantation procedure. Aspects of this pre-operative information may be digitized and entered into the CP 70 (Figs. 5 and 6). One example of pre-operative information that may be provided to the CP includes information acquired during the patient's trial stimulation phase. For example, the location of the trial leads and the electrodes that provided the best pain relief for the patient may be collected and entered and may provide a reasonable prediction of a desired placement for the permanent leads. Fluoroscopic images of the trial leads (or information gleaned therefrom) may be entered, as well as the identity and location of the trial lead electrodes used during the trial stimulation phase.

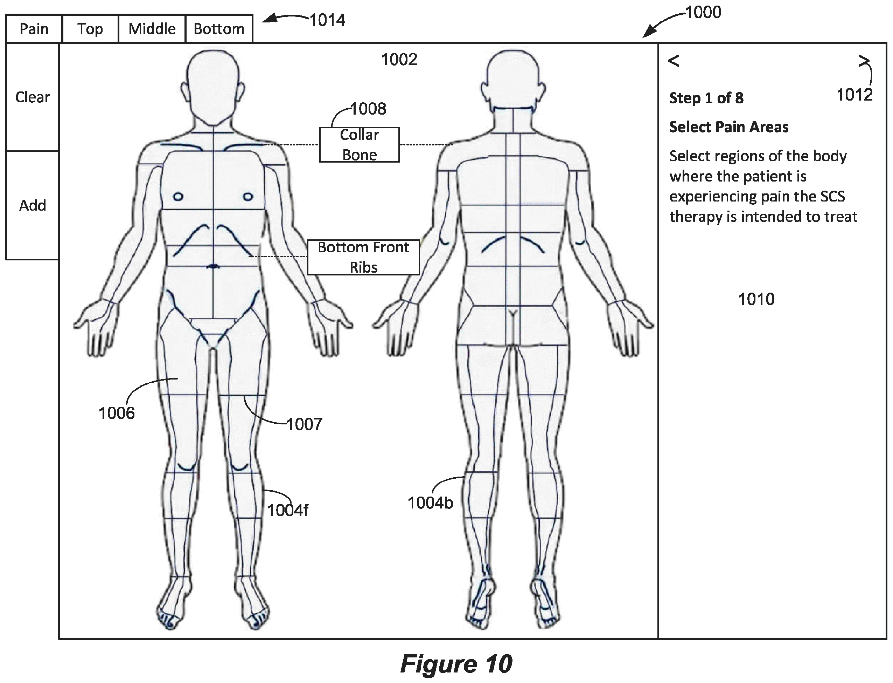

Other pre-operative information that may be entered into the CP may include information relating to the location of the patient's pain, and specifically, pain that can be targeted/treated by the stimulation therapy. The information relating to the location of the patient's pain may include information regarding affected dermatomes and other anatomic structures. Some embodiments of the disclosed systems may comprise GUIs or other means of inputting pain location information, translating that information into data that can be used by the system, as well as digitizing that data. For example, U.S. Provisional Application Serial No. 2026/0007898 ("the 898 Publication), describes body maps that allow a patient and/or clinician to indicate body locations where the patient feels pain and/or feels paresthesia. Figure 10 illustrates an example of an embodiment of a GUI 1000. Such GUIs may be provided on a screen of the CP or another computing device whereby collected data may be transferred to the CP. The GUI 1000 comprises a body map area 1002 that depicts body maps 1004f and 1004b, representing the front and back of the patient, respectively. The body maps 1004f and 1004 b may be divided into selectable body map regions 1006, which may correspond to intuitive body locations that a user (e.g., the patient and/or the clinician) can use to select the pain locations for the patient. For example, the labeled body map region 1006 corresponds to the right upper thigh. Generally, the body maps may include any number of body map regions 1006, providing varying degrees of granularity for selecting the pain locations. For example, the body map may contain 100 or more selectable body map regions. In some embodiments the body map regions may be tessellated or otherwise divided into smaller, or more granulated selectable regions. According to some embodiments, the GUI may provide multiple sets of body maps having different configurations of body map regions. This may allow a clinician to select a particular set of body maps for a patient depending on the patient's indications, for example. As one example, if a patient is being treated for diabetic neuropathy, the clinician may select a body map that provides more granularity (i.e., more body map regions) in the foot region.

In the illustrated body maps the selectable body map regions 1006 are demarcated by body map region boundaries 1007 that are visible to the user. In other embodiments, the body map region boundaries may not be apparent to the user. In such embodiments, the user may select the body map region or location by coloring or drawing on an anatomical location and underlying logic and/or operations correlate the user's "selection" to particular body map regions, though that correlation may, or may not, be visible to the user. As used herein, the terms "select," "selectable," etc. refer to the ability to mark or select a location on a body map, for example, using any of the selection tools described in more detail below.

The body maps may also include anatomical landmarks 1008 to aid in the identification of the relevant body locations. Two anatomical landmarks are shown in the illustration, but the GUI 1000 typically includes more (which are omitted for clarity). In some embodiments, the identification of the anatomical landmarks may be listed, as shown in the illustration. The landmark indicators may be toggled on and off in some embodiments. In other embodiments, the labels may appear when the user hovers their cursor over the landmark, for example. The GUI may comprise a text panel 1010 that may be configured to guide the user while collecting the relevant information. For example, the text panel 1010 may provide step-by-step instructions for executing the data collection. The instruction panel may include navigation elements 1012 for navigating through various steps of the data collection. The GUI may comprise selector elements 1014 for navigating through the various functionalities of the GUI.

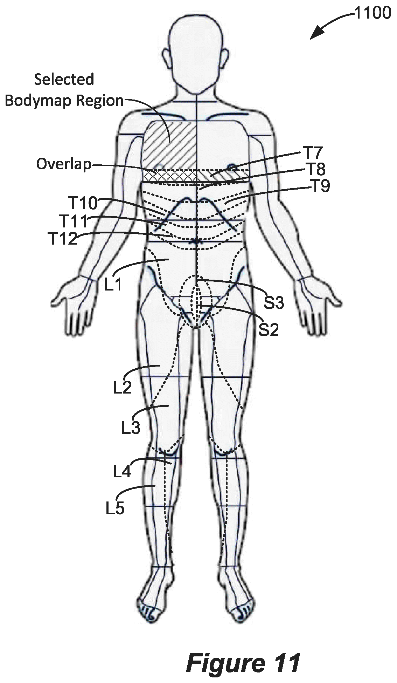

Once the patient or clinician has provided their pain location(s) to the GUI, the system can map those pain locations anatomically relevant data that the CP can use during the lead placement procedure. Examples of such anatomically relevant data include data specifying affected dermatomes, vertebral levels, medio/lateral coordinates, underlying neural anatomy, muscular anatomy, etc. Embodiments of the pain location system may include an underlying translation layer, as described in the above-incorporated '898 Publication. The translation algorithm determines the extent of overlap of the selected body map regions of the body map region-based maps with representations of the various underlying dermatomes or other anatomical structures. For example, assume that in the body map region-based body map 1100 shown in Figure 11, the user has selected the right chest body map region as an area where pain is experienced. That body map region may comprise a given number of pixels or grid cells. As used herein, the pixel may refer to any type of grid cell. The body map may be projected onto a map of dermatomes, some of which are illustrated in the body map 1100, for illustrative purposes. Each of the dermatomes in the dermatome map may also comprise a given number of pixels or grid cells. Notice that the selected body map region overlaps a portion of the T7 dermatome. The extent of overlap between the selected body map region and the underlying dermatome may be used to determine if the T7 dermatome corresponds to a vertebral level associated with pain. For example, the algorithm may determine the number of pixels of the selected body map regions that overlap with pixels of the T7 dermatome. This information can be used by the lead placement algorithm to suggest a trial lead placement position, as well as to provide the clinician with an indication of where to place the non-spinal transducers, as described further below.

Referring again to Figure 9, during the pre-operative phase various acceptance criteria 910 may be collected and provided to the system. Examples of such acceptance criteria may include the identity of dermatomes that should be modulated by the stimulation, and which typically correspond to the location of the patient's pain and, in some embodiments, surrounding dermatomes. For example, the acceptance criteria may relate to a percentage of targeted dermatomes, and/or a sufficient amount of each/all dermatomes. In some embodiments, the acceptance criteria may comprise or relate to a sufficient/acceptable/minimum required pain relief will be achieved. Other acceptance criteria may comprise a specification of an ideal placement of the leads with respect to the neural elements of the spinal cord neural elements that are to be stimulated. In other words, the clinician may specify how they want the leads to be placed with respect to the target neural elements. For example, the clinician may endeavor to place the leads such that electrodes on a certain location on the lead (e.g., the top half, the middle third, etc.) may be used to stimulate the target neural elements. This may be specified based on the clinician's evaluation of the possibility of post-operative lead migration. For example, if stimulating the target neural elements requires using the topmost electrodes on the lead, stimulation may be compromised if the lead migrates caudally over time. Accordingly, the clinician may seek to place the lead so that electrode near the middle of the lead will primarily be used to provide stimulation. In some embodiments, the clinician may seek to place the leads in a location that is best fit for a particular type or modality of stimulation, for example, a location that is fit for stimulation of a particular type, e.g. field shape (monopole, bipole, tripole, etc) and/or pattern (tonic, burst, modulating, pseudo-random).

The intra-operative phase 904 of the guidance method may comprise locating/placing the non-spinal transducers at the correct location(s) upon the patient's body (Step 912). Generally, at least some of the transducers will be placed at the dermatomes for which the spinal stimulation will be targeted, typically where the patient experiences pain. In some embodiments, the clinician may use the information gleaned from the pre-operative determinations of pain location(s) 908 to inform the placement of the transducers (for example, using the body maps discussed above). For example, a GUI on the CP may provide indicia of proper transducer placement, for example, using a body map with markers indicating where to place the transducer(s). In some embodiments, locations for transducer placement may be projected onto the patient's body or displayed on an external screen (i. e., one that is not a part of the CP).

As mentioned above, the purpose of the transducers is to measure how stimulation at various locations in the spinal cord modulates neural signals impacting the pain locations. Thus, step 914 of the intra-operative lead placement guidance methods involves placing the lead array at an initial trial location, using various of the stimulation electrodes to apply stimulation to the patient's spinal cord, and recording physiological responses using the non-spinal transducers. In some embodiments, the guidance system may suggest an initial lead placement based on the pre-operative information discussed above. For example, a GUI of the CP may display an indicator, such as a heatmap, lead shadow or outline, or other visual cue of a suggested initial lead placement. As mentioned, the system may derive the initial lead placement based on information gleaned from the location of the patient's trial lead(s) during the pre-operative trial phase, the location of the patient's pain/affected dermatomes, etc. The suggestions may be derived from population-level analysis as an initial guess. For example, it is commonly accepted that leads placed from T8-T10 may be effective for targeting lower back and leg pain. Likewise, the vertebral/medio-lateral mapping described above in the context of the body maps 1000/1100 (Figs. 10 and 11) may provide guidance for the suggested initial lead placement.

Once the lead(s)/electrode array 17 is placed an initial trial location, test stimulation may be applied to the spinal cord at one or more pre-determined electrodes/locations upon the electrode array (still step 914). For example, test stimulation may be provided at the top (rostral) left and right corners of the electrode array and the middle. According to some embodiments, the system may be configured to provide test stimulation using a preset intra-operative mapping sweep, whereby specific electrodes/locations are pre-selected to sequentially provide stimulation and physiological data (discussed below) is automatically collected after each test stimulation. The stimulation program may be defined based on the lead placement (visualized or otherwise confirmed in the system), impedance differences between the electrodes, and/or the patient's pain regions. An example of a mapping sweep algorithm is described in more detail below.

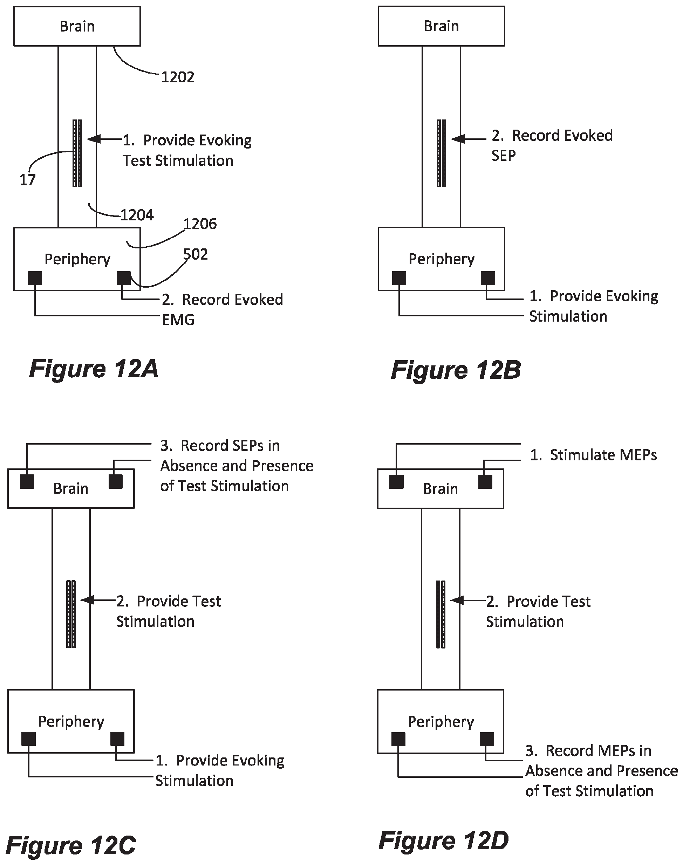

Figures 12A - 12D schematically illustrate some embodiments of physiological measurements that may be used to determine how spinal stimulation modulates neural elements within the patient's spinal cord, which modulation effects physiological recordings at the monitored pain locations and/or the transmission of neural signals between the pain locations and the patient's brain. Each of the illustrations depict a schematic representation of the patient's brain 1202, spinal cord 1204, and peripheral locations 1206. The lead array 17 is illustrated in the patient's spinal cord and the peripherally located non-spinal transducers 502 are placed at the locations determined in step 912 of the workflow 900 (Fig. 9).

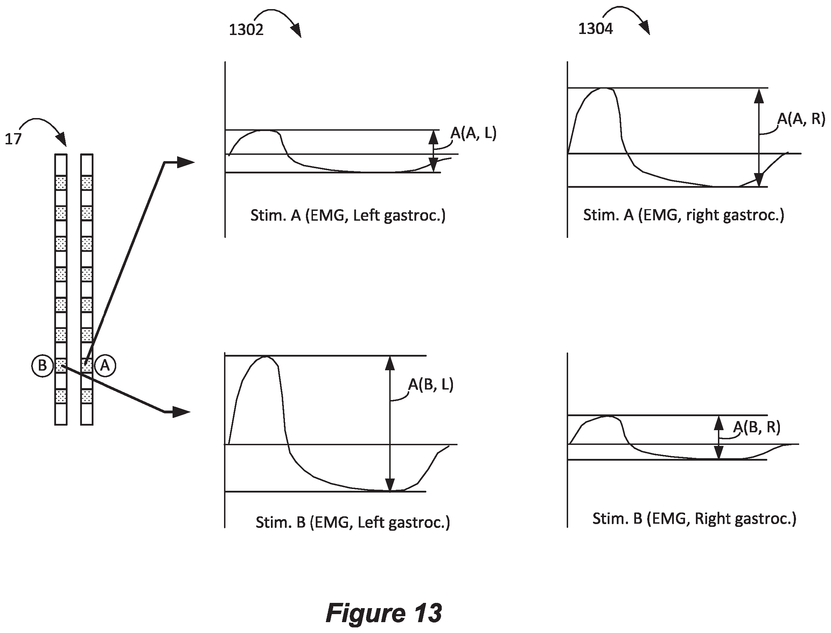

In the embodiment illustrated in Figure 12A, test stimulation is applied to the patient's spinal cord using the predetermined locations/electrodes of the electrode array 17. If the amplitude of the stimulation is sufficient, the stimulation may evoke efferent electrical signals that may be detected in the peripheral nerves and muscles that are innervated by the motor nerves originating from the location being stimulated at the spinal cord. Those signals may be recorded, for example, using electromyography (EMG) electrodes, which are placed at the targeted dermatomes. The EMG electrodes are configured to probe for signals that are synchronized (or slightly delayed) with respect to the applied test stimulation. Generally, the stimulation amplitude required to evoke such motor signals may be greater than that of therapeutic stimulation that would be comfortable for the patient, where the patient awake. Such high stimulation amplitudes may generate electric fields in the spinal cord that are large enough such that stimulation multiple different contacts may activate the same muscles. For example, Figure 13 illustrates EMG signals recorded on the left and right side of a patient's body when stimulation is applied to the patient's spinal cord using spinal electrodes located on the left vs. the right side of an electrode array 17. The EMG signals are recorded at the patient's left and right gastrocnemius (calf muscle). When stimulation is applied at the electrode labeled A on the right side of the array a relatively small EMG signal 1302 is observed on the left side of the patient's body and a larger EMG signal 1304 is observed on the right side. That is, the amplitude A(A, R) is greater than the amplitude A(A, L). This indicates that the stimulation on the right side of the lead primarily elicits responses on the right side of the body, but the electric field is large enough to also elicit a response on the left side of the body. The situation is reversed when stimulation is provided at the electrode labeled B on the left side of the electrode array 17. That is, the amplitude A(B, L) of the EMG recorded at the patient's left calf is greater than the amplitude A(B, R) recorded at the patient's right calf. In some embodiments, the system may be configured to use the amplitudes of the evoked responses, including the differences between the responses evoked by stimulation at the various array locations, to map the electrodes of the array to locations on the patient's spinal cord that target particular dermatomes of the patient's body. The differences of the amplitudes may be weighted and used to determine stimulation amplitudes and fractionalizations that can be supplied to the spinal electrodes to achieve approximate midline activation, for example.

As shown in Figures 12A and 13, recording EMG signals at the patient's periphery which are evoked by stimulation at different locations at the patient's spinal cord may indicate the mediolateral location of the electrode array and how the positions of the various electrodes map to evoked EMG signals recorded at the various tested dermatomes. The guidance system executed in the CP may be configured with a mapping algorithm that uses this information to suggest locations on the patient's spinal cord that would be expected to treat pain at the affected dermatomes. The mapping algorithm may be based on understood or inferred organization of the spinal cord. For example, if stimulation at a certain vertebral level elicits EMG signals at the patient's calf, then the mapping algorithm (again, based on known spinal cord anatomy) may suggest that stimulation 1 mm above or below that vertebral level would be expected to be therapeutic, for example, based known relationships of efferent and afferent neural elements in the spinal cord.

Figure 12B illustrates another example of a physiological measurement that may be used to guide electrode placement. This example assumes that the ETS is configurable to sense neural potentials at the patient's spinal cord as discussed above with reference to Figure 7. In this example, electrical stimulation is provided at stimulation electrodes placed at the patient's periphery, for example, at the targeted dermatomes/pain locations. The peripheral electrodes may be EMG electrodes, needle electrodes, dermal electrodes, transcutaneous electrodes, or the like. Sufficient electrical stimulation evokes afferent neural signals that travel to the patient's spinal cord where they may be recorded using the spinal electrodes of the array 17. Thus, the electrodes where the evoked signals are detected can be correlated to the dermatomal source of the stimulation.

Figure 12C illustrates another example wherein stimulation is provided is provided at peripheral electrodes. The peripheral stimulation is configured to evoke sensory evoked potentials (SEPs) that may be sensed/recorded at the patient's somatosensory cortex using transducers configured on the patient's scalp, for example. In some embodiments, the evoking peripheral stimulation may be applied to the target dermatome(s)/pain location(s) in the absence of any spinal stimulation to obtain a baseline recording of SEPs. Then the evoking peripheral stimulation may be applied while trolling stimulation is provided using spinal electrodes of the array 17. This can indicate which stimulation locations at the patient's spinal cord modulate the conduction of the SEPs to the patient's brain.

Figure 12D illustrates another example, wherein stimulation is provided to the patient's motor cortex, for example, using transcranial magnetic stimulation (TMS) or transcranial electrical stimulation, to elicit motor evoked potentials (MEPs). The MEPs may be sensed/recorded at peripheral electrodes/transducers located at the targeted dermatome(s)/pain location(s). In some embodiments, the stimulation evoking the MEPs may be asserted first in the absence of any spinal stimulation to obtain a baseline recording of MEPs. Then the evoking cortex stimulation may be applied while trolling stimulation is provided using spinal electrodes of the array 17. This can indicate which stimulation locations at the patient's spinal cord modulates the conduction of the MEPs to the patient's target dermatome(s)/pain location(s).

Now that examples of the various physiological measurements have been discussed, embodiments of the preset intra-operative mapping sweep mentioned above may be described. According to some embodiments, the ETS and/or the CP may be configured with such a mapping sweep algorithm to facilitate the trolling of various electrodes of the spinal electrode array and the collection of the physiological responses, and to map electrode locations to locations on the patient's body responding to the spinal stimulation. In one example, assume that the electrode array comprises a paddle lead or parallel percutaneous leads configured such that the rostral electrodes of each lead are at equal rostral/caudal heights. Stimulation (e.g., cathodic stimulation) may be delivered using a given set of electrode configurations, e.g., the top row of electrodes, the middle row, and the bottom row in succession. The stimulation delivered at each row may begin sweeping from an amplitude of 0 mA up to a preset maximum amplitude (e.g., 5 mA, which may be increased/decreased as necessary). The amplitudes may increase in stepwise increments (0.5 mA, for example). In some embodiments, the user may choose to skip remaining higher amplitudes once results are achieved at a lower amplitude. In some embodiments, each amplitude may be delivered for a preset number of pulses (e.g., 10 pulses) before proceeding to the next amplitude. The first muscle, dermatome, body location, etc. to show a physiological response to the stimulation at the lowest amplitude may be mapped to that electrode configuration. Once a set number of muscles (or other locations, dermatomes, etc.) have responded, the mapping sweep may skip to the next fractionalization on the lead. If at the end of the sweep a muscle, dermatome, or other location is not covered by stimulation (or only covered at higher amplitude stimulation), logic, such as the following, may be instantiated: (1) the lowest area of the body and highest area of the body at which a response was recorded will be noted; (2) if the region of interest is physically below the lowest region of the array, then both rows up from the bottom will be stimulated as above; (3) if the region of interest is physically above the highest region-(3a) if the region of interest is the buttocks or below, the two rows below the top will be stimulated as above, if the stimulation fails to cover the below regions, the lead should be moved down and testing at the lower levels repeated; (3b) if the region of interest is above the buttocks (e.g., the back), this region can be hard to capture, if the top electrode activated buttocks and legs at significantly higher amplitudes than it took the middle and lower fractionalization to activate their respective muscles, then it can be assumed that the back should be covered; otherwise it may be assumed that the back is not covered and the lead should be moved upwards and testing at the higher levels should be repeated. In the event that the electrodes are not parallel and not equidistant from each other, the fractionalization of the stimulation applied may be configured to mimic stimulation at the top, middle, and bottom rows, adjusting fractionalizations such that the relative penetration of the majority of the charge is approximately equivalent at each stimulation location.

Referring again to Figure 9, once physiological measurements have been made when the electrode array is positioned in an initial trial position (step 914), the guidance algorithm/system may use the physiological measurements to recommend steps to reposition the array/lead(s) (step 916). The steps of (re)positioning the lead (step 914) and recommending further repositioning (step 916) may be performed repeatedly and iteratively until the acceptance criteria discussed above are met. The system uses the physiological measurements (i.e., EMGs, SEPs, MEPs, etc.) to determine which body locations/dermatomes are being modulated by the spinal cord stimulation and can map the stimulating electrodes to those locations. In other words, for each test stimulation applied using spinal electrodes of the electrode array, the guidance system knows: (1) the present position of the electrode array/leads (e.g., from present fluoroscopy scans); (2) which electrodes of the array are activated to apply the test stimulation; and (3) which body locations/dermatomes are being modulated by that test stimulation. The guidance algorithm can be configured to evaluate this information with respect to the acceptance criteria. For example, the guidance algorithm may determine if the targeted pain locations/dermatomes are sufficiently covered by stimulation provided using the tested electrodes in the array. The algorithm may determine if the selected electrodes that do cover the targeted locations/dermatomes are positioned at the desired locations within the array according to the acceptance criteria. For example, if the electrodes covering the targeted dermatomes are located at the extreme top or bottom of the array, the algorithm may recommend repositioning the array to meet the acceptance criteria.

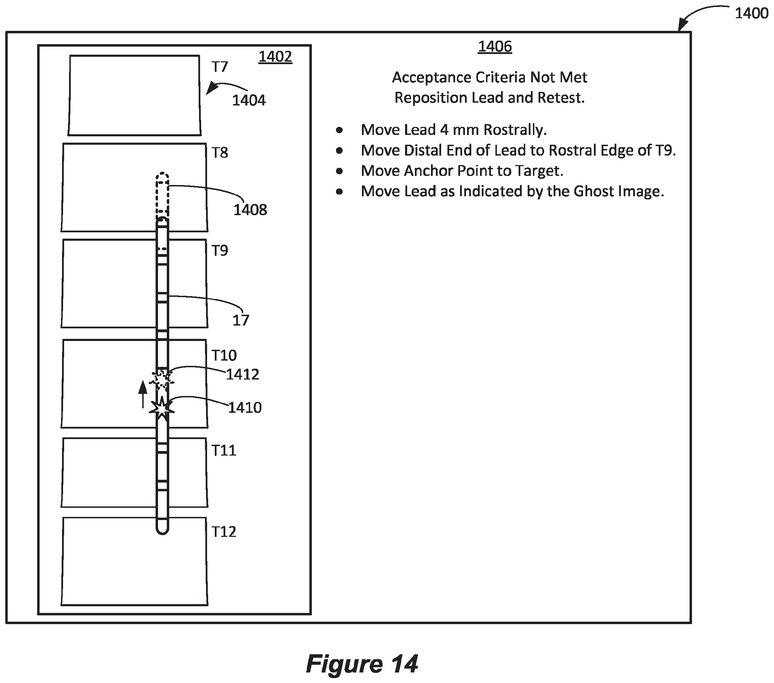

If the acceptance criteria are not met, then the guidance algorithm may be configured to recommend repositioning the spinal array/leads and retesting the new position. The guidance may be based on understood and/or inferred organization of the spinal column. For example, if the stimulation at the lead's/array's present position is activating dermatomes/locations below the targeted dermatomes/locations, then the algorithm would typically instruct the clinician to relocate the lead in a rostral direction. In some embodiments, the algorithm may use a GUI on the CP to provide an indication of where to reposition the lead, for example, a direction and a distance to move the lead. Figure 14 illustrates features of an embodiment of a relocation GUI 1400. The GUI may be configured to display a fluoroscopic image 1402 (or an image derived therefrom), which may show the present location of the lead(s)/array 17 and spinal structures, such as vertebrae 1404. The GUI may also include a dialogue section 1406 or other means of communicating information to the clinician during the lead placement operation. The dialogue section may provide instructions for repositioning the lead. For example, it may instruct the clinician to move the lead a certain distance and a certain direction. The GUI may reference anatomical landmarks, for example, landmarks that are visible or represented in the fluoroscopic image. In the illustrated example the dialogue box has suggested repositioning the lead/array with reference to a particular vertebra (T9). In some embodiments, the GUI may use augmented reality elements, such as a "ghost image" or target image 1408 that may be projected onto the fluoroscopic image illustrating the target placement for the lead. As another example, the GUI may project an indicator, such as an anchor point 1410 upon the image of the lead, and instruct the clinician to reposition the lead so that the anchor point coincides with a target 1412, which may be projected on the fluoroscopic image. Though not shown in the illustration, the GUI may also provide information regarding which dermatomes/body locations are responsive to the various test stimuli. The GUI may display a representation of overlap or convergence of the test stimuli and the target(s), for example, as a Venn diagram, heatmap, or the like. In some embodiments, the GUI may display an indication of a confidence of good outcome, confidence of outcome after the recommended move, population % of good outcome at present location vs. with the move, or some other indication of a benefit or expected outcome based on making the recommended move.

As mentioned above, the sequence of (re)positioning the lead and acquiring test measurements may be repeated iteratively. In some embodiments, the guidance system/algorithm may be adaptive. For example, in early sequences the algorithm may recommend small lead movements, but upon further data acquisition larger lead movements may be recommended. In some embodiments, the guidance system/algorithm may employ Bayesian methods to guide the testing/retesting and lead movement recommendations. Examples of such methods include knowledge gradient, multi-armed bandit, stochastic gradient descent, and the like.

Referring again to Figure 9, the sequence of (re)positioning the lead/acquiring test measurements (914) and recommending additional lead movements (916) may be repeated until the tested lead position yields physiological measurements that meet the acceptance criteria to a predetermined specification set in the system or to the clinician's satisfaction. At that point, the permanent leads may be secured in place, and the implantation procedure may be continued (step 918). For example, the IPG may be surgically implanted and connected to the lead(s)/array, etc.

In some embodiments, the information gathered during the intra-operative lead positioning procedure concerning the electrode contacts within the lead/array that provided the best activation/modulation of the targeted body locations/dermatomes may be saved and used to inform later post-operative programming of the patient's IPG (920). As is known in the art an issue in stimulation therapy, and SCS therapy in particular, is determining the stimulation parameters that are best able to treat a patient's symptoms. As noted above, SCS stimulation parameters can include features like the amplitude of stimulation (I), the pulse width (PW) of the stimulation pulses, and the frequency (F) at which the stimulation pulses are issued. Other stimulation parameters used to place the stimulation at a location (L) in the electrode array 17 to best treat the patient's symptoms are also important. This location L is determined by the electrodes that are active to form the stimulation, the polarity of those active electrodes, and a percentage indicating a relative amount of the amplitude each active electrode should receive. As the electrodes that are capable of modulating the patient's target dermatomes/body locations are known and saved from the intra-operative implantation procedure, according to the disclosed methods and systems, those electrode locations provide a starting point for subsequent post-operative programming.

It is also recognized in the art that an SCS patient's IPG may be programmed with multiple stimulation programs that are hoped to treat the patient's symptoms under different conditions, pain levels, activity levels, and according to the patient's preferences. The patient may be able to select from among a menu of different stimulation programs, for example, using their external controller 60 (Fig. 4). For example, some of the patient's stimulation programs may provide stimulation that the patient can feel, i.e., that evokes paresthesia. Other programs may be sub-perception. Some stimulation programs may be configured to target neural elements in the dorsal horn of the spinal cord whereas others may be configured to target dorsal column fibers. Examples of a variety of stimulation programs are discussed in U.S. Non-Provisional Patent Application No. 19/446,508, filed January 12, 2026. The post-operative programming and "fitting" of such programs are typically performed using a CP 70, as described above. In some embodiments, the CP 70 used for post-operative programming may be configured to receive the data from the intra-operative procedures described above and use that data to suggest and pre-populate parameters for such stimulation programs. Such pre-populated stimulation programs may be further optimized during the fitting procedure.

The embodiments described herein are primarily focused on SCS. But it will be recognized by a person of skill in the art that the methods and systems may be used in other contexts of neural stimulation, for example, in peripheral nerve stimulation.

## Claims

1. A system for assisting the surgical implantation of an electrode array in the spinal column of a patient for delivering therapeutic stimulation to the patient's spinal cord to treat pain in the patient, wherein the electrode array comprises a plurality of spinal electrodes, the system comprising:
a user interface,
control circuitry configured to:
receive an indication of locations of one or more peripheral transducers placed at peripheral locations on the patient's body,
receive one or more acceptance criteria,
cause a predetermined one or more of the plurality of spinal electrodes to provide test spinal stimulation to the patient's spinal cord,
receive one or more signals indicative of neural activity, wherein the neural activity is either evoked or recorded using the one or more peripheral transducers,
determine a modulation of the neural activity by the test spinal stimulation,
use the modulation to determine if the acceptance criteria is met,
if the acceptance criteria is met, cause the user interface to display an indication that the acceptance criteria is met,
if the acceptance criteria is not met, use the modulation to determine a suggested movement of the electrode array and cause the user interface to display an indication of the suggested movement.

2. The system of claim 1, wherein the one or more acceptance criteria comprises one or more of a desired location within the electrode array selected for delivering the therapeutic stimulation, an acceptable coverage of body locations targeted for the therapeutic stimulation, and an acceptable coverage of dermatomes targeted for the therapeutic stimulation.

3. The system of any one of claims 1 or 2, wherein the neural activity comprises potentials evoked by the test spinal stimulation and recorded by the one or more peripheral transducers.

4. The system of claim 1, wherein the neural activity comprises motor evoked potentials (MEPs) evoked by stimulating the patient's motor cortex and recorded by the one or more peripheral transducers.

5. The system of claim 4, wherein determining a modulation of the neural activity by the test spinal stimulation comprises:
recording first MEPs in the absence of the test spinal stimulation,
recording second MEPs in the presence of the test spinal stimulation, and
determining a difference between the first and second MEPs.

6. The system of claim 1, wherein the neural activity comprises sensory evoked potentials (SEPs) evoked by stimulation provided by the one or more peripheral transducers and recorded at the patient's somatosensory cortex.

7. The system of claim 6, wherein determining a modulation of the neural activity by the test spinal stimulation comprises:
recording first SEPs in the absence of the test spinal stimulation,
recording second SEPs in the presence of the test spinal stimulation, and
determining a difference between the first and second SEPs.

8. The system of claim 1, wherein the neural activity comprises afferent neural signals evoked by stimulation provided by the one or more peripheral transducers and recorded using one or more of the spinal electrodes.

9. The system of any one of claims 1-8, wherein displaying an indication of the suggested movement comprises displaying an image indicative of the present location of the electrode array and an indication of a position the electrode array will occupy following the suggested array movement.

10. The system of any one of claims 1-9, wherein displaying an indication of the suggested movement comprises displaying a distance and direction to move the array.

11. The system of any one of claims 1-10, wherein the control circuitry is configured to cause the user interface to display an indication of an initial location for the electrode array.

12. The system of claim 11, wherein the control circuitry is configured to receive one or more parameters derived from a previously placed trial electrode array.

13. The system of any one of claims 1-12, wherein the control circuitry is configured to cause the user interface to display indicia of body locations where the peripheral transducers should be placed.

14. The system of claim 13, wherein the system is configured to:
receive one or more user pain inputs indicating locations on the patient's body that the patient feels pain,
digitize the pain inputs, and
use the digitized inputs to generate the indicia of body locations where the peripheral transducers should be placed.

15. The system of claim 13, wherein the system is configured to:
display one or more body maps comprising selectable body map regions,
receive indications of one or more selected body map regions corresponding to body locations where the patient feels the pain,
use a translation algorithm to determine mediolateral locations and dermatomes where the patient feels the pain based on the selected body map regions, and
use the mediolateral locations and dermatomes to determine the indicia of body locations where the peripheral transducers should be placed.
